Europäisches Patentamt

European Patent Office

Office européen des brevets

(11) Publication number: **0 405 299 A2**

# EUROPEAN PATENT APPLICATION

(21) Application number: 90111530.3

(22) Date of filing: **19.06.90**

(51) Int. Cl.5: **A61K 31/60**, A61K 47/08, A61K 9/70

(30) Priority: **23.06.89 IT 2098489**

(43) Date of publication of application:
**02.01.91 Bulletin 91/01**

(84) Designated Contracting States:
**CH DE ES FR GB IT LI SE**

(71) Applicant: **De Benedittis, Giuseppe**
**Via Carbonera, 21**
**I-20137 Milan(IT)**

(72) Inventor: **De Benedittis, Giuseppe**
**Via Carbonera, 21**
**I-20137 Milan(IT)**

(74) Representative: **Gervasi, Gemma et al**
**NOTARBARTOLO & GERVASI Srl Viale**
**Bianca Maria 33 33**
**I-20122 Milan(IT)**

(54) Composition for the treatment of acute herpetic neuralgia and post-herpetic neuralgia.

(57) There is described a composition for the topic treatment of Acute Herpetic Neuralgia (AHN) and Post-Herpetic Neuralgia (PHN) consisting in an association between NSAD or ALD drugs and ethyl ether.

EP 0 405 299 A2

## COMPOSITION FOR THE TREATMENT OF ACUTE HERPETIC NEURALGIA AND POST-HERPETIC NEURAL-GIA.

### Field of the invention

The present invention relates to a composition containing a NSAD drug (Non-Steroid Antiinflammatory Drugs) or an ALD drug (Aspirin-Like Drugs) in association with ethyl ether for the topic treatment of Acute Herpetic Neuralgia and of Post-Herpetic Neuralgia.

### Background of the invention

The great clinical importance, from the algogenic viewpoint, of Acute Herpetic Neuralgia (AHN) and of its more fearful sequence, the Post-Herpetic Neuralgia (PHN), resides in the growing diffusion of the herpetic infection, which tends to affect by far more precocious age ranges than the pre-senile and the senile ones - usually affected - and in its well-known refractoriness to the commonest forms of antalgic treatment.

By consequence, these is a constant search for drugs, which could permit to efficaciously fight such affections.

### Detailed description of the invention

The present invention relates to a composition comprising non-steroid antiinflammatory drugs (NSAD) or acetylsalicylic-like drugs (ALD) and ethyl ether, which composition has proved to be effective in the topic treatment of Acute Herpetic Neuralgia (AHN) and of Post-Herpetic Neuralgia (PHN), with particular reference to the pain control.

According to a preferred embodiment, the compositions of the present invention consist of acetylsalicylic acid in the form of a very fine powder solvo-suspended in ethyl ether. Preferably, the ether/ASA ratio ranges from 2% to 10% v/w. The new compositions have the appearance of a lactescent, more or less thick suspension, which is directly applied to the part.

Equally and surprisingly excellent results are obtained, for example, also with indomethacin or with Naproxen or, as already said, with any NSAD or ALD.

The amount of the various antiinflammatory drugs to be utilized in the preparation of the new compositions are equianalgesic amounts with respect to acetylsalicylic acid. The ether amount to be used shall be always such as to provide a thick, topically applicable solution/suspension.

### Experimental part

750-1500 mg of acetylsalicylic acid or equianalgesic doses of ALD or NSAD (said doses being variable as a function of the extension of the hyperesthetic skin area dermatomically affected by the herpetic neuralgia or post-herpetic neuralgia) were triturated in a small metal or ceramic (not plastic material) cup by means of a pestle in order to form a very fine powder. 20-30 ml of ethyl ether were added and the whole was stirred. Acetylsalicylic acid partially dissolves in the ethyl ether, partially remains in lactescent suspension and partially remains as a separate solid. A gauze pad was saturated with the solution/suspension, which was uniformly spread on the whole hyperesthetic skin area. The ether volatilizes very quickly, while the acetylsalicylic acid powder settles as a fine whitish nubecula onto the interested dermatomers. Areas, if any, left uncoated by the first spreading had to be retouched by means of a second spreading until complete coating of the area affected by the herpes.

The antalgic effect tends to appear very quickly, usually in a few minutes from the application, and reaches its maximum after about 20-30 minutes. The duration of the effect is relatively variable and comprised between 2 and 8 hours, the average being of 4-6 hours.

The best results were obtained with an average of three-four applications per day.

As already cited hereinbefore, the above-mentioned effect can be achieved only through the original association between ethyl ether and acetylsalicylic acid or an equivalent analgesic drugs. It can be assumed that ethyl ether, by virtue of its high lipophilia, facilitates the cutaneous absorption of the drug and a more intimate contact with the cutaneous receptors, at concentration levels difficult to be reached with other administration modalities.

However, it remains to be explained why the same effect or analogous effects are not obtainable with other solvents capable of giving solutions/suspensions with aspirin or other analgesic drugs.

Clinical results

The topic treatment with the new compositions based on acetylsalicylic acid was preliminarily carried out on 15 patients affected by Acute Herpetic Neuralgia and Post-Herpetic Neuralgia. Seven patients (six females and one male) of age ranging from 26 and 77 years were treated in the acute phase (within 2 months from the beginning of the cutaneous eruption and of the associated pain). The treatment site was the thoracodorsal in all cases. The pain intensity, measured by VAS (visual analog system) was comprised between 4.5 and 6.8 (average = 5.9).

Eight additional patients (six females and two males) of age ranging from 56 to 82 years were treated, who had been suffering from a Post-Herpetic Neuralgia for a period varying from 6 to 84 months. In three patients the localization was craniofacial, in four patients thoracodorsal and in the last patient lumbar. The pain intensity (measured by means of VAS) was comprised between 1.0 and 8.0.

The treatment results were classified as follows:

(A): excellent (pain average reduction by 75%-100% with respect to the basic line)

(B): good (reduction by 50%-75%)

(C): fairly good (reduction by 25%-50%)

(D): poor/none (reduction below 25%).

Six out of seven patients suffering from Acute Herpetic Neuralgia exhibited excellent results, while a patient exhibited a fairly good result. Only one patient out of seven developed a post-herpetic neuralgia (against an average normal incidence of 30% reported in literature).

In the patients with post-herpetic neuralgia, excellent results were obtained in two cases, good results in further 2 cases, poor or no results in the remaining four cases. In the aggregate, the positive results (excellent/good) interested 50% of the treated sample.

A further clinical testing making use of the new compositions based on acetylsalicylic acid was conducted on 36 patients, 24 of whom were affected by Acute Herpetic Neuralgia (AHN) and 12 by Post-Herpetic Neuralgia (PHN).

The obtained results are recorded in Table 1, in which the classification has the meaning defined hereinbefore.

TABLE 1

| CLINICAL RESULTS | | | | |
|---|---|---|---|---|
| Syndrome | Excellent | Good | Fairly good | Poor/None |
| AHN (n = 24) | 18 (75.0%) | 4 (16.7%) | 2 (8.3%) | ---- |
| PHN (n = 12) | 3 (25.0%) | 4 (33.3%) | ---- | 5 (41.7%) |

Although no immunologic controls were carried out, it is important to point out that only one case out of 24 in the acute phase (4%) (however, moderately positive to the treatment) had developed the fearful sequence of PHN.

No collateral effect or topic complication or systemic complication were observed in the course of the testing conducted with the compositions based on acetylsalicylic acid. Conversely, the dermatologic observation revealed a significant reduction (4-7 days instead of the usual 14-21 days) in the desiccation and resolution times of the AHN cutaneous phenomena.

In another testing, 8 patients affected by Acute Herpetic Neuralgia and 3 patients affected by Post-Herpetic Neuralgia were subjected to a blind treatment with randomized application of placebo (absence of active drug), indomethacin (100 mg), piroxicam (20 mg) and dichlophenac (100 mg) in ethyl ether solution.

In none of the treated cases, the placebo exhibited an antalgic activity while indomethacin, piroxicam and dichlophenac exerted an antalgic activity significantly not dissimilar to the one exhibited by acetylsalicylic acid.

3

However, in the two cases of treatment with piroxicam and in one case of treatment with indomethacin, cutaneous reactions developed.

The use of the new compositions according to the present invention can be considered as a valid alternative to more complicated and doubtfully effective treatments practised so far.

## Claims

1. A composition for topic application, consisting of a solution/suspension of non-steroid antiinflammatory drugs or of aspirin-like drugs (referred to as NSAD and ALD, respectively) in ethyl ether.

2. The composition according to claim 1, wherein the antiinflammatory drug is acetylsalicylic acid.

3. The composition according to claim 2, wherein the ether/AAS ratio ranges from 2% to 10% v/w.

4. A gauze pad, impregnated with a composition according to claims 1,2 and 3.

5, A method for therapeutically treating patients affected by acute herpetic neuralgia or by post-herpetic neuralgia, wherein the hyperesthetic skin area is spread with a composition consisting of a solution/suspension of an antiinflammatory drug NSAD or ALD in ethyl ether.